# EUROPEAN PATENT APPLICATION

(11) **EP 4 571 305 A1**
(43) Date of publication of application: **18.06.2025**
(21) Application number: 24215470.6
(22) Date of filing: 26.11.2024
(51) Int. Cl.: G01N 33/49, B01L 3/00, G01N 15/1433, G06T 7/00, G03H 1/04

(54) **SYSTEMS AND METHODS TO MEASURE OCCLUSION OF BLOOD CELLS IN MICROFLUIDIC CHANNELS**

(30) Priority: 15.12.2023 US 202318542096
(71) Applicant: Honeywell International Inc., Charlotte, NC 28202 (US)
(72) Inventor: BROWN, Andy W., Charlotte, 28202 (US); SHAFAI, Moin S., Charlotte, 28202 (US); ZAKARIA, Ryadh A., Charlotte, 28202 (US); WILLIAMS, Evelyn Kendall, Charlotte, 28202 (US); FIBBEN, Kirby, Charlotte, 28202 (US); LAM, Wilbur, Charlotte, 28202 (US)
(74) Representative: Haseltine Lake Kempner LLP

(57) **Abstract**

A system and a method for measuring occlusion of blood cells flowing in microfluidic channels is disclosed. The system includes an occlusion device having one or more microfluidic channels with a predefined cross-sectional area. The one or more microfluidic channels are configured to allow flow of a plurality of blood cells of a blood sample within an interior surface of the one or more microfluidic channels. Further, at least one imager is configured to generate one or more digital holography images or videos of the plurality of blood cells transiting the one or more microfluidic channels. Further, at least one processor is configured to receive the one or more digital holography images or videos and analyze the generated one or more digital holography images or videos to quantify and/or characterize occlusions formed within the one or more microfluidic channels.

## Description

### TECHNOLOGICAL FIELD

Example embodiments of the present disclosure relate generally to a diagnostic device, and more particularly, to systems and methods that utilize microfluidic channels and an inline digital holography technique to measure occlusion of blood cells and to determine severity of a hematologic disease.

### BACKGROUND

Hematologic diseases such as Sickle Cell Disease (SCD) require early diagnosis and constant monitoring and treatment throughout a patient's lifespan. About 3 million people worldwide suffer from the SCD, mostly in Africa, India and the Middle East, with an estimated 100,000 affected in the U.S., according to the Centers for Disease Control and Prevention. SCD affects 1 in 375 African American newborns born in the U.S. Early diagnosis through newborn screening, followed by simple interventions, has dramatically reduced SCD related mortality in the US. More than 800 children are born with SCD every day in Africa, and more than half of the children die in childhood due to lack of diagnosis and early treatment. Effective treatment of SCD still remains a challenge in preventing childhood and adult mortality, especially in the developing world due to requirements of skilled individuals and the high cost of currently available modalities. Frequent SCD severity monitoring presents insurmountable challenges due to a lack of objective measures of the disease state and the reliance on highly subjective patient-reported symptoms like pain.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a summary of some example embodiments to provide a basic understanding of some aspects of the present disclosure. This summary is not an extensive overview and is intended to neither identify key or critical elements nor delineate the scope of such elements. It will also be appreciated that the scope of the disclosure encompasses many potential embodiments in addition to those here summarized, some of which will be further described in the detailed description that is presented later.

In an example embodiment, a system is disclosed. The system comprises an occlusion device having one or more microfluidic channels with a predefined cross-sectional area. The one or more microfluidic channels are configured to allow flow of plurality of blood cells of a blood sample within an interior surface of the one or more microfluidic channels. The system further includes at least one imager configured to generate one or more digital holography images or videos of the plurality of blood cells transiting the one or more microfluidic channels. The system further includes at least one processor that is operationally coupled to the at least one imager. The at least one processor is configured to receive the one or more digital holography images or videos and analyze the generated one or more digital holography images or videos to quantify or characterize occlusions formed within the one or more microfluidic channels.

In some embodiments, the predefined cross-sectional area of the one or more microfluidic channels corresponds to cross-sectional area of small blood vessels.

In some embodiments, the interior surface of the one or more microfluidic channels is coated with a plurality of endothelial cells.

In some embodiments, the one or more microfluidic channels comprises at least one inlet and an outlet. The at least one inlet receives the blood sample and the outlet allows to discharge the plurality of blood cells through the one or more microfluidic channels.

In some embodiments, a lensless in-line digital holography configuration is used for imaging.

In some embodiments, the at least one processor is configured to analyze the generated one or more digital holography images or videos using Artificial Intelligence/Machine Learning module to detect presence of occluded channels within the one or more microfluidic channels.

In some embodiments, to quantify or characterize occlusions of the occluded channels within the one or more microfluidic channels includes generating a score or relative indicator of a health status or indicating an efficacy of a medical treatment of a user based on a severity of a hematologic disease in the plurality of blood cells. In some embodiments, the plurality of blood cells are unstained and untagged.

In some embodiments, at least one user device is configured to receive a report related to the health status.

In another example embodiment, a method is disclosed. The method comprises steps of allowing a plurality of blood cells of a blood sample to flow within one or more microfluidic channels having a predefined cross-sectional area. The one or more microfluidic channels are configured to allow flow of the plurality of blood cells of the blood sample within an interior surface of the one or more microfluidic channels. The method further includes generating, via at least one imager, one or more digital holography images or videos of the plurality of blood cells transiting the one or more microfluidic channels; and analyzing, via at least one processor, the generated one or more digital holography images or videos to quantify, characterize, or quantify and characterize occlusions formed within the one or more microfluidic channels.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a block diagram of an exemplary system to determine severity of a hematologic disease in accordance with an example embodiment of the present disclosure;
FIG. 2A illustrates a perspective view of an exemplary imaging device for determining severity of the hematologic disease in accordance with an example embodiment of the present disclosure;
FIG. 2B illustrates an enlarged view of the exemplary imaging device in accordance with an example embodiment of the present disclosure;
FIG. 3 illustrates an exemplary occlusion device with a microfluidic channel in accordance with an example embodiment of the present disclosure;
FIG. 4A illustrates the exemplary occlusion device with the one or more microfluidic channels having multiple dimensions at various points in accordance with an example embodiment of the present disclosure;
FIG. 4B illustrates an enlarged view of the exemplary occlusion device with the one or more microfluidic channels having multiple dimensions at various points in accordance with an example embodiment of the present disclosure;
FIG. 4C illustrates an example of holographic microscopy images of a normal blood sample and a hematologic sample occluded within the one or more microfluidic channels in accordance with an example embodiment of the present disclosure;
FIG. 5 illustrates an example holographic microscopy image with blood sample cells flowing from an inlet to an outlet of the one or more microfluidic channels in accordance with an example embodiment of the present disclosure;
FIG. 6 illustrates a flowchart of a method to determine severity of the hematologic disease i.e. a sickle cell disease in accordance with an example embodiment of the present disclosure; and
FIG. 7 illustrates an exemplary scenario of the system to determine severity of the hematologic disease in accordance with an example embodiment of the present disclosure.

### DETAILED DESCRIPTION

Some embodiments will now be described more fully hereinafter with reference to the accompanying drawings, in which some, but not all, embodiments of the present disclosure are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of systems and methods to detect occlusion of SCD blood cells in microfluidic channels. Embodiments may allow blood cells of a blood sample to flow within one or more microfluidic channels having a predefined cross-sectional area. Embodiments may generate one or more digital holography images or videos of the blood cells transiting the one or more microfluidic channels. Embodiments may analyze the generated one or more digital holography images or videos to quantify and/or characterize one or more occlusions formed within the one or more microfluidic channels. Thereafter, a report related to user's health status is generated to show severity of a hematologic disease i.e., sickle cell disease (SCD) in the plurality of blood cells.

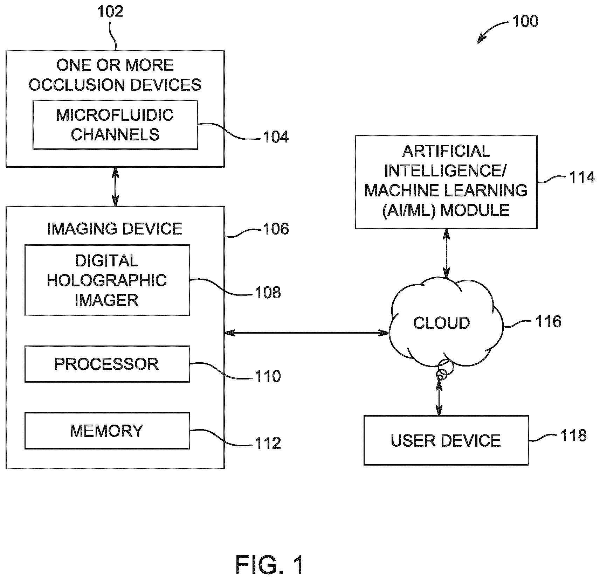

FIG. 1 illustrates a block diagram of a system 100 to determine severity of a hematologic disease, in accordance with an example embodiment of the present disclosure.

The system 100 may comprise an occlusion device 102 having one or more microfluidic channels 104 and an imaging device 106. The imaging device 106 may comprise at least one imager 108 and at least one processor 110 operated on stored instructions in a memory 112. The one or more microfluidic channels 104 may have a predefined cross-sectional area. Further, the predefined cross-sectional area of the one or more microfluidic channels 104 may correspond to cross-sectional area of small blood vessels (not shown). The one or more microfluidic channels 104 may be configured to allow flow of a plurality of blood cells of a blood sample within an interior surface (not shown) of the one or more microfluidic channels 104. In some embodiments, the interior surface of the one or more microfluidic channels 104 may be coated with a plurality of endothelial cells. In some other embodiments, the interior surface of the one or more microfluidic channels 104 may be coated using Laminin or p-selectin. In some example embodiments, various other coating options may exist depending on which pharma medication is being evaluated for efficacy, without departing from the scope of the disclosure. Hereinafter, the occlusion device 102 and one or more occlusion devices 102 may be used interchangeably.

In some embodiments, the at least one imager 108 may be configured to generate one or more digital holography images or videos of the plurality of blood cells transiting the one or more microfluidic channels 104. The at least one imager 108 may be referred to as a digital holographic imager. In some embodiments, the at least one imager 108 may use a lensless in-line digital holography configuration to generate the one or more digital holography images or videos. In some embodiments, the at least one processor 110 may utilize angular spectrum propagation for modelling the propagation of a wave field to generate the one or more digital holography images or videos. It may be noted that the angular spectrum propagation may be configured to computationally focus the one or more digital holography images or videos, without departing from the scope of the disclosure.

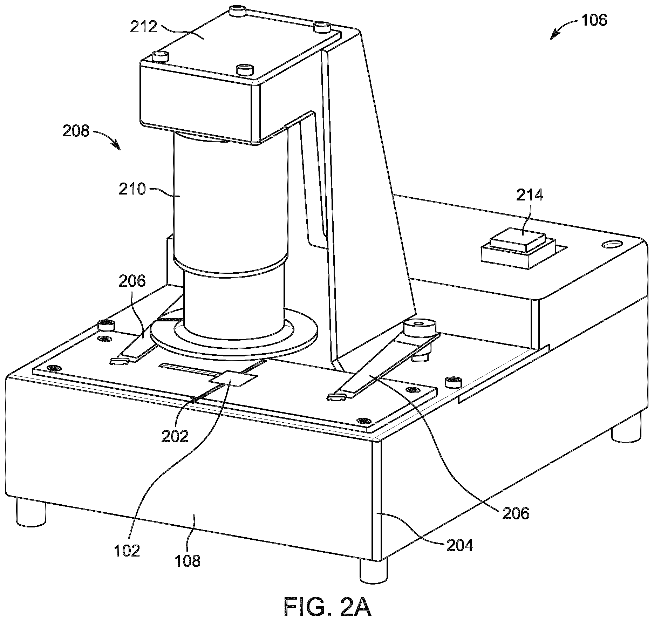

The at least one processor 110 may be operationally coupled to the at least one imager 108. The at least one processor 110 may be configured to receive the one or more digital holography images or videos. The at least one processor 110 may be configured to analyze the generated one or more digital holography images or videos to determine severity of the hematologic disease. In some embodiments, the hematologic disease may correspond to diseases relating to blood cells such as, RBCs, Leukocytes, Platelet, etc. In some embodiments, the hematologic disease may correspond to sickle cell disease. The detailed working of the at least one processor 110 may be described in greater detail in conjunction with FIGS. 2A-4C.

In some embodiments, the at least one processor 110 may include suitable logic, circuitry, and/or interfaces that are operable to execute one or more instructions stored in a memory 112 to perform predetermined operations. In one embodiment, the at least one processor 110 may be configured to decode and execute any instructions received from one or more other electronic devices or server(s). The at least one processor 110 may be configured to execute one or more computer-readable program instructions, such as program instructions to carry out any of the functions described in this description. Further, the processor may be implemented using one or more processor technologies known in the art. Examples of the processor include, but are not limited to, one or more general purpose processors (e.g., INTEL^{®} or Advanced Micro Devices^{®} (AMD) microprocessors) and/or one or more special purpose processors (e.g., digital signal processors or Xilinx^{®} System On Chip (SOC) Field Programmable Gate Array (FPGA) processor).

Further, the memory 112 may be communicatively coupled to the at least one processor 110. In some embodiments, the memory 112 may be configured to store a set of instructions and data executed by the at least one processor 110. Further, the memory 112 may include the one or more instructions that are executable by the at least one processor 110 to perform specific operations. It is apparent to one skilled in the art that the one or more instructions stored in the memory 112 enable the hardware of the system 100 to perform the predetermined operations. Some of the commonly known memory implementations include, but are not limited to, fixed (hard) drives, magnetic tape, floppy diskettes, optical disks, Compact Disc Read-Only Memories (CD-ROMs), and magneto-optical disks, semiconductor memories, such as ROMs, Random Access Memories (RAMs), Programmable Read-Only Memories (PROMs), Erasable PROMs (EPROMs), Electrically Erasable PROMs (EEPROMs), flash memory, magnetic or optical cards, or other type of media/machine-readable medium suitable for storing electronic instructions.

Further, the system 100 may comprise an Artificial Intelligence/Machine Learning (AI/ML) module 114 communicatively coupled to the at least one processor 110, via a cloud 116. The at least one processor 110 along with the AI/ML module 114 may analyze the generated one or more digital holography images or videos to quantify and/or characterize one or more occlusions formed within the one or more microfluidic channels 104. In various embodiments, the quantification and/or characterization may be performed by determining a percentage of occluded channels at some point of time during perfusion and a rate at which the one or more microfluidic channels 104 may be occluded. Such quantification and/or characterization of the occlusions within the one or more microfluidic channels 104 may be configured to generate a score or relative indicator of a user's health status or indicate an efficacy of a medical treatment of the user based on a severity of the hematologic disease in the plurality of blood cells. In some embodiments, the AI/ML module 114 may be configured to detect and classify individual cells and/or groups of cells. The classification may ensure that the detected cells are the plurality of blood cells and not some other noise or dust/debris. In some alternate embodiments, the AI/ML module 114 may be integrated within the at least one processor 110 to analyze the generated one or more digital holography images or videos without the need for remotely monitoring via the cloud 116. In some embodiments, the AI/ML module 114 may segregate the blood cells from other elements present in the image.

In some embodiments, the cloud 116 may facilitate a communication link between the AI/ML module 114 and the at least one processor 110. It may be noted that the cloud 116 may be referred as a network interface that facilitates a communication link among the other components of the system 100. Further, the cloud 116 may be a wireless network and/or a wired network. The cloud 116 may be implemented using one or more communication techniques. The one or more communication techniques may be Radio waves, Wi-Fi, Bluetooth, ZigBee, Z-wave and other communication techniques, known in the art.

The system 100 may further include at least one user device 118 that is configured to receive the quantified occlusion of the plurality of blood cells to display a report related to the user's health status. The at least one user device 118 may be wired to the at least one processor 110 or may be coupled, via the cloud 116. In some embodiments, the at least one user device 118 may include a wired or wireless devices operationally coupled to the system 100. For example, a desktop or laptop computer, any tablet, smart phone, or other devices known in the art.

Further, the system 100 may include an input/output circuitry (not shown) that may enable a user to communicate or interface with the system 100 via the at least one user device 118. In some example embodiments, the at least one user device 118 may include a control room computer system or other portable electronic devices. It may be noted that the input/output circuitry may act as a medium transmit input from the at least one user device 118 to and from the system 100. In some embodiments, the input/output circuitry may refer to the hardware and software components that facilitate the exchange of information between the user and the system 100. In one example, the system 100 may include a graphical user interface (GUI) (not shown) as an input circuitry to allow the user to input data via the at least one user device 118. The input/output circuitry may include various input devices such as keyboards, barcode scanners, GUI for the user to provide data and various output devices such as displays, printers for the user to receive data. In another example, the input/output circuitry may include various output circuitry such as indicators to indicate the correct and incorrect placement of the occlusion device 102.

In some alternate embodiments, the system 100 may include a communication circuitry (not shown). The communication circuitry may allow the imaging device 106 and the at least one user device 118 to exchange data or information with other systems. Further, the communication circuitry may include network interfaces, protocols, and software modules responsible for sending and receiving data or information. In some embodiments, the communication circuitry may include Ethernet ports, Wi-Fi adapters, or communication protocols like HTTP or MQTT for connecting with other systems. The communication circuitry may allow the system 100 to stay up-to-date and accurately track levels of blood samples/cells occluded within the one or more microfluidic channels 104.

It will be apparent to one skilled in the art the above-mentioned components of the system 100 have been provided only for illustration purposes. In some embodiments, the system 100 may include other components as well, without departing from the scope of the disclosure.

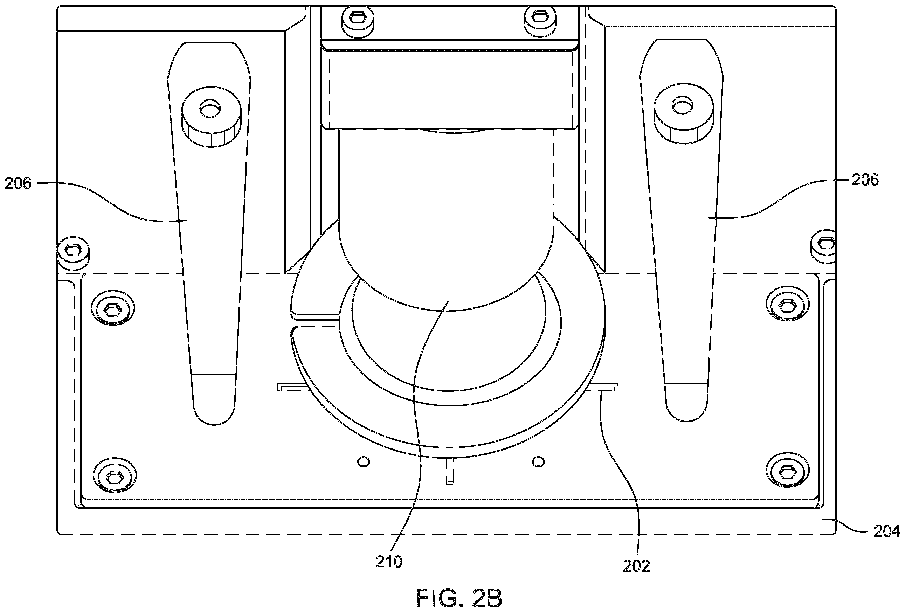

FIG. 2A illustrates a perspective view of the imaging device 106 for determining severity of the hematologic disease, in accordance with an example embodiment of the present disclosure. FIG. 2B illustrates an enlarged view of the imaging device 106, in accordance with an example embodiment of the present disclosure. FIGS. 2A-2B are described in conjunction with FIG. 1.

The imaging device 106 may comprise a cross hair 202 positioned above a housing 204. In some embodiments, the occlusion device 102 having the one or more microfluidic channels 104 may be placed on the cross hair 202 of the housing 204. The one or more microfluidic channels 104 may be positioned firmly on the cross hair 202 using one or more clamps 206. In some embodiments, the one or more clamps 206 may correspond to metal plates that may be rotatably fixed at one end and free at other end. The free end may be slide onto the one or more microfluidic channels 104 once placed over the housing 204. The one or more microfluidic channels 104 placed on the cross hair 202 may be occluded or blocked by the plurality of blood cells of the blood sample on the interior surface of the one or more microfluidic channels 104. In some embodiments, the interior surface of the one or more microfluidic channels 104 may be coated with a plurality of endothelial cells. It may be noted that the one or more microfluidic channels 104 may be placed at any other position of the housing 204 as well, without departing from the scope of the disclosure.

As discussed above, the imaging device 106 may comprise the at least one imager 108 and the at least one processor 110. Further, the at least one imager 108 may be configured to generate the one or more digital holography images or videos of the occluded channels in the one or more microfluidic channels 104. Further, the at least one imager 108 may be referred to as a digital holographic imager. In some embodiments, the at least one imager 108 may use a lensless in-line digital holography configuration to generate the one or more digital holography images or videos.

The imaging device 106 may comprise an illumination source 208. The illumination source 208 may be directed towards the one or more microfluidic channels 104. The illumination source 208 may be configured to facilitate the at least one imager 108 to generate the one or more digital holography images or videos. In some embodiments, the illumination source 208 may comprise a height adjustable light tube 210 and a laser driver 212 that may be installed above the at least one imager 108. The height adjustable light tube 210 may be a telescopic structure with adjustment of height from the one or more microfluidic channels 104 placed on the cross hair 202 of the housing 204. Further, the imaging device 106 may be provided with a switch 214 to control the laser driver 212 and thereby, the illumination source 208. In various embodiments, the height adjustable light tube 210 and the laser driver 212 may together be referred to as the illumination source 208. In some embodiments, the illumination source 208 may be positioned at a height, e.g., 8 centimeters (cm) from the occlusion device 102, such that the plurality of blood cells inside the one or more microfluidic channels 104 may receive a uniform laser beam.

Further, the at least one processor 110 may be communicatively coupled to the at least one imager 108. The at least one processor 110 may be configured to receive the one or more digital holography images or videos. Further, the at least one processor 110 may analyze the generated one or more digital holography images or videos to quantify and/or characterize one or more occluded channels within the one or more microfluidic channels 104. In one example embodiment, the occluded channels may be quantified and/or characterized to generate a score or relative indicator of the health status and indicate the efficacy of a medical treatment of the user based on the severity of the hematologic disease. It may be noted that the user herein refers to a patient or a person having a medical condition. In one example embodiment, the score or relative indicator may include one or more reference ranges to allow for quick indication of the severity of the hematologic disease. In some embodiments, the score may include one or more reference ranges to allow for quick indication of the severity of a sickle cell disease (SCD).

In some embodiments, the at least one processor 110 may be configured to analyze the generated one or more digital holography images or videos using the AI/ML module 114. In some embodiments, the generated one or more digital holography images or videos may be analyzed to count the occluded channels within the one or more microfluidic channels 104. It may be noted that the count indicates the severity of the hematologic disease in the plurality of blood cells.

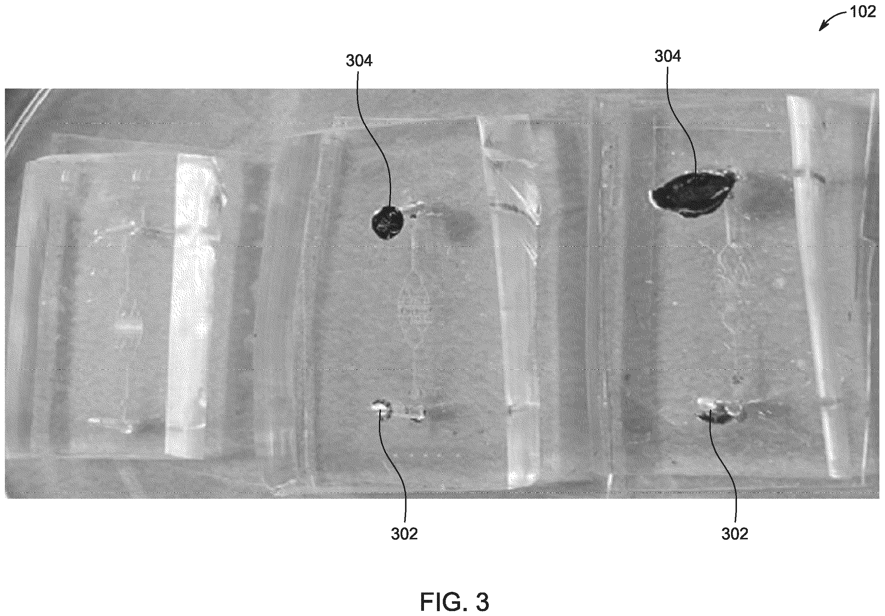

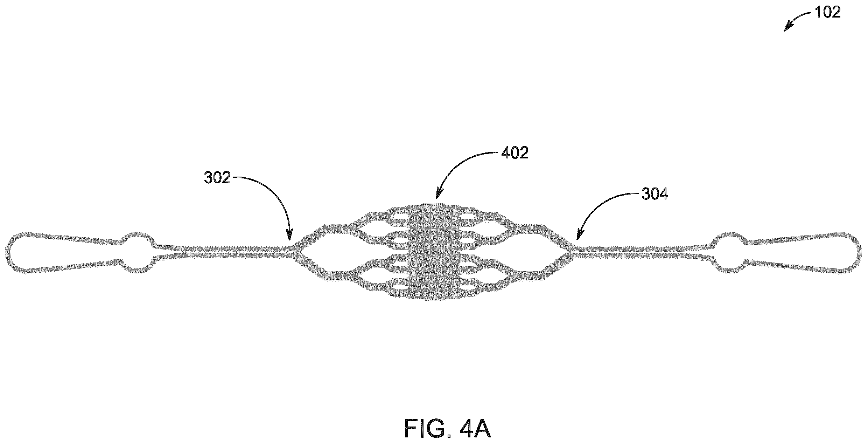

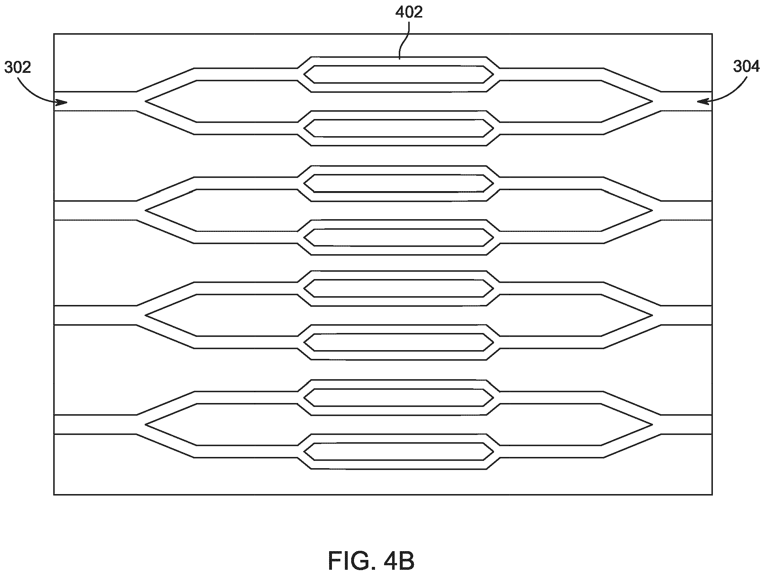

FIG. 3 illustrates the occlusion device 102 with the one or more microfluidic channels 104, in accordance with an example embodiment of the present disclosure. FIG. 4A illustrates the occlusion device 102 with the one or more microfluidic channels 104 having multiple dimensions at various points, in accordance with an example embodiment of the present disclosure. FIG. 4B illustrates an enlarged view of the occlusion device 102 with the one or more microfluidic channels 104 having multiple dimensions at various points, in accordance with an example embodiment of the present disclosure. FIGS. 3-4B are described in conjunction with FIGS. 1-2B.

As illustrated in FIG. 3, each of the one or more microfluidic channels 104 of the occlusion device 102 may have at least one inlet 302 and an outlet 304. The one or more microfluidic channels 104 may have a branched structure 402, as illustrated in FIG. 4A, between the at least one inlet 302 and the outlet 304. The one or more microfluidic channels 104 may have a predefined cross-sectional area at the various points across the branched structure 402. The one or more microfluidic channels 104 may be configured to facilitate flow of the plurality of blood cells of the blood sample within the interior surface of the one or more microfluidic channels 104. It may be noted that the plurality of blood cells may pass from the at least one inlet 302 to the outlet 304 via the branched structure 402. The branched structure 402 may have a small cross-section as compared to the at least one inlet 302.

In some example embodiments, the one or more microfluidic channels 104 may have dimensions of 30x30 micrometer (µm) that may be sufficiently large to permit the dynamic interaction of RBCs, WBCs, platelets, and other blood components that together form occlusions. In some embodiments, the blood sample cells may flow with a flow rate. In one example, the flow rate may include a flow rate of approximately 1 µL/minute. In some embodiments, a constant pressure instead of a constant flow rate may be used. Stated differently, a healthy blood may flow between the at least one inlet 302 and the outlet 304 in a suitable time frame without forming clots or getting occluded in the branched structure 402. The suitable time frame may correspond to few seconds or several minutes depending upon the flow velocity of the blood sample. In some example embodiments, a microfluidic channel may have one or more dimensions such as width and length in micrometer (µm) 30x500, 43x300, and 61x300, and height 30 micrometer. It will be apparent to one skilled in the art that the above-mentioned dimensions have been provided only for illustration purposes, without departing from the scope of the disclosure.

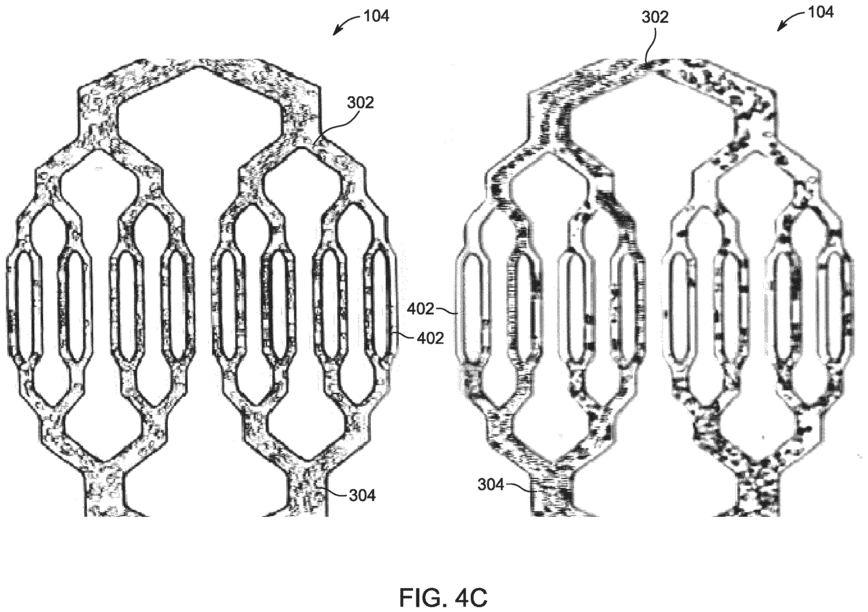

FIG. 4C illustrates an example of holographic microscopy images of a normal blood sample and a hematologic diseased sample occluded within the one or more microfluidic channels 104, in accordance with an example embodiment of the present disclosure. FIG. 4C is described in conjunction with FIGS. 1-4B.

As illustrated in FIG. 4C, a normal blood sample may easily flow between the at least one inlet 302 towards the outlet 304, via the branched structure 402. The plurality of blood cells, i.e., the blood cells in the normal blood sample may not occlude or clot within the branched structure 402. Further, the SCD blood sample when perfused from the at least one inlet 302 may be occluded or may clot within the branched structure 402. As discussed above, the one or more digital holography images captured by the at least one imager 108 may confirm occlusion of the SCD blood sample within the branched structure 402 of the one or more microfluidic channels 104.

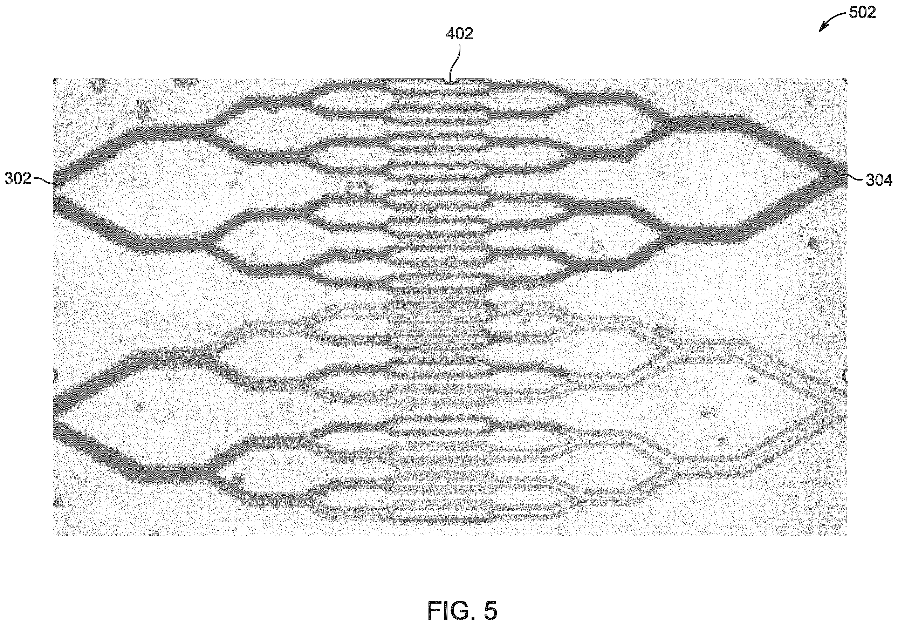

FIG. 5 illustrates an example holographic microscopy image 502 with blood sample cells flowing from the at least one inlet 302 to the outlet 304 of the one or more microfluidic channels 104, in accordance with an example embodiment of the present disclosure. FIG. 5 is described in conjunction with FIGS. 1-4C.

The one or more microfluidic channels 104 may be analyzed by the digital holography microscopic image 502 of the suitable time frame, for the normal blood sample and the SCD blood sample, as illustrated in FIG. 4C. In one example, the at least one imager 108 may capture a video showing flow of blood sample from the at least one inlet 302 towards the outlet 304. In another example, the at least one imager 108 may capture one or more images within the suitable time frame showing flow of blood sample from the at least one inlet 302 towards the outlet 304. It will be apparent that the flow of the blood sample from the at least one inlet 302 towards the outlet 304 varies according to the severity of the blood sample. The SCD blood sample may get occluded within the branched structure 402, while the normal blood sample may pass easily towards the outlet 304, as illustrated in FIG. 4C.

As discussed, the at least one processor 110 may be configured to analyze the generated one or more holography images or videos using the AI/ML module 114 to detect the presence of occluded microfluidic channels 104. Further, the quantification and/or characterization of the occluded microfluidic channels 104 may be configured to generate a score or relative indicator of the health status and/or indicate the efficacy of the medical treatment of the user based on the severity of a hematologic disease in the plurality of blood cells.

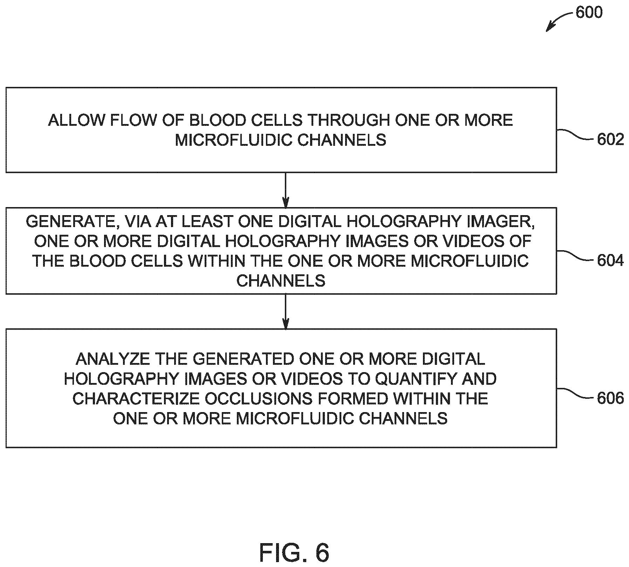

FIG. 6 illustrates a flowchart of a method 600 to determine severity of the hematologic disease, in accordance with an example embodiment of the present disclosure. FIG. 6 is described in conjunction with FIGS. 1-5

At operation 602, the plurality of blood cells of the blood sample are allowed to flow within one or more microfluidic channels having a predefined cross-sectional area. The predefined cross-sectional area of the one or more microfluidic channels 104 may correspond to cross-sectional area of small blood vessels. The interior surface of the one or more microfluidic channels 104 may be coated with the plurality of endothelial cells.

At operation 604, the at least one imager 108 generates one or more digital holography images or videos of the plurality of blood cells transiting the one or more microfluidic channels 104. In some embodiments, the at least one imager 108 may use the lensless in-line digital holography configuration to generate the one or more digital holography images or videos.

At operation 606, analyze the generated one or more digital holography images or videos to quantify and/or characterize occlusions formed within the one or more microfluidic channels 104. In some embodiments, the at least one processor 110 may reconstruct the generated one or more digital holography images or videos. Further, the at least one processor 110 may analyze the reconstructed one or more digital holography images or videos using the AI/ML module 114 to quantify and/or characterize the occlusions of blood cells from the plurality of blood cells formed within the one or more microfluidic channels 104, and thereby detects the presence of occluded microfluidic channels. 104. In some embodiments, the occlusions of the plurality of blood cells are quantified to generate the score or relative indicator of the health status or indicate the efficacy of the medical treatment of the user based on a severity of the hematologic disease in the plurality of blood cells.

It will be appreciated that the method 600 may be implemented by one or more the embodiments disclosed herein, which may be combined or modified as desired or needed. Additionally, the steps in the method 600 may be modified, changed in order, performed differently, performed sequentially, concurrently or simultaneously, or otherwise modified as desired or needed.

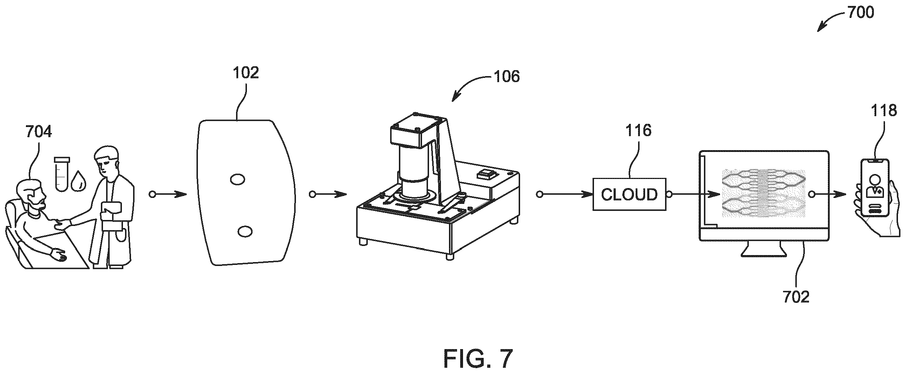

FIG. 7 illustrates an exemplary scenario 700 of the system 100 to determine severity of the hematologic disease, in accordance with an example embodiment of the present disclosure. FIG. 7 is described in conjunction with FIGS. 1-6.

In some embodiments, the exemplary scenario 700 may comprise the imaging device 106 as discussed above in conjunction with FIGS. 1-5. Further, the system 100 may comprise the at least one user device 118 that may be configured to receive the quantified occlusion of the plurality of blood cells to display a report on the at least one user device 118 related to a user's 704 health status. Further, the report may be displayed on a display device 702. In some example, embodiments, the at least one user device 118 and the display device 702 may include a smartphone, a tablet, a laptop, a personal computer (PC), a smart watch or any other device known in the art to display the report. In one example, the user 704 may receive the report in his/her user device, like the smartphone. In some embodiments, the report may comprise at least one score or relative indicator of the user's 704 health status and/or indicate an efficacy of a medical treatment of the user 704 based on the severity of the hematologic disease in the plurality of blood cells. The system 100 may further transfer the report to the user 704, via the cloud 116. In some embodiments, the AI/ML module 114 may be implemented over the cloud 116 to interpret the data of the report entailing the details of the user's 704 health status.

As discussed above, the at least one processor 110 may be configured to analyze the generated one or more digital holography images or videos using AI/ML module 114. The AI/ML module 114 may be configured to count total occluded channels after the plurality of blood cells pass through the one or more microfluidic channels 104 of the occlusion device 102. The AI/ML module 114 may implement AI/ML algorithms to quantify occlusion formed within the one or more microfluidic channels 104. The AI/ML module 114 may count total number of locations, and temporal durations of occlusions in the one or more microfluidic channels 104 to determine severity of the hematologic disease.

In some embodiments, the total number of occluded channels may be a proxy measurement for the severity of the SCD. The quantification and/or characterization of the occluded microfluidic channels 104 may be configured to generate the score or relative indicator of the user 704 health status and/or indicate an efficacy of the medical treatment of the user based on the severity of a hematologic disease in the plurality of blood cells.

In some embodiments, total number of occluded channels, location within the branched structure 402, and/or temporal duration of the occlusion may be proxy measurements for the severity of hematologic diseases such as, a sickle cell disease. It may be noted that the dynamic and interactive nature of the biophysical flow characteristics of all components of the blood sample may be evaluated. It may also be noted that the occlusion may be caused by combinations of cell stickiness/adhesion from the components of the blood sample, i.e., hemoglobin content, morphological changes cell stiffness/deformability and other parameters working together within the one or more microfluidic channels 104. It will be apparent to one skilled in the art that the one or more digital holography images or videos may be acquired one or more times to identify occlusions and characterize the occluded sample, to determine severity of the hematologic diseases.

The present disclosure utilizing microfluidic channels and a digital holography technique to determine severity of the hematologic diseases like sickle cell disease. In some embodiments, the microfluidic channels may be coated/functionalized in order to cause diseased blood cells to preferentially occlude the microfluidic channels. The images/videos are analyzed to quantify occlusion of the microfluidic channels. Therefore, a detailed report of severity of hematologic disease, such as sickle cell disease, is provided to the user remotely. The present disclosure also provides a benefit of not labelling the cells, that is, the cells are unstained and untagged. This provides a cost benefit of determining the severity of diseases without using fluorescent dyes for tagging the cells.

Many modifications and other embodiments of the disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A system comprising:
an occlusion device comprising one or more microfluidic channels with a predefined cross-sectional area, wherein the one or more microfluidic channels are configured to allow flow of plurality of blood cells of a blood sample within an interior surface of the one or more microfluidic channels;
at least one imager configured to generate one or more digital holography images or videos of the plurality of blood cells transiting the one or more microfluidic channels; and
at least one processor operationally coupled to the at least one imager and configured to:
receive the one or more digital holography images or videos; and
analyze the generated one or more digital holography images or videos to quantify or characterize occlusions formed within the one or more microfluidic channels.

2. The system of claim 1, wherein the predefined cross-sectional area of the one or more microfluidic channels corresponds to cross-sectional area of small blood vessels.

3. The system of claim 1, wherein the interior surface of the one or more microfluidic channels is coated with a plurality of endothelial cells.

4. The system of claim 1, wherein the one or more microfluidic channels comprises at least one inlet and an outlet.

5. The system of claim 4, wherein the at least one inlet receives the blood sample and the outlet allows to discharge the plurality of blood cells through the one or more microfluidic channels.

6. The system of claim 1, wherein a lensless in-line digital holography configuration is used for imaging.

7. The system of claim 1, wherein the at least one processor is configured to analyze the generated one or more digital holography images or videos using Artificial Intelligence/Machine Learning module to detect presence of occluded channels within the one or more microfluidic channels.

8. The system of claim 7, wherein to quantify or characterize occlusions of the occluded channels within the one or more microfluidic channels includes generating a score or relative indicator of a health status or indicating an efficacy of a medical treatment of a user based on a severity of a hematologic disease in the plurality of blood cells.

9. The system of claim 1, wherein the plurality of blood cells are unstained and untagged.

10. A method comprising:
allowing a plurality of blood cells of a blood sample to flow within one or more microfluidic channels having a predefined cross-sectional area, wherein the one or more microfluidic channels are configured to allow flow of the plurality of blood cells of the blood sample within an interior surface of the one or more microfluidic channels;
generating, via at least one imager, one or more digital holography images or videos of the plurality of blood cells transiting the one or more microfluidic channels; and
analyzing, via at least one processor, the generated one or more digital holography images or videos to quantify, characterize, or quantify and characterize occlusions formed within the one or more microfluidic channels.

11. The method of claim 10, wherein the predefined cross-sectional area of the one or more microfluidic channels corresponds to cross-sectional area of small blood vessels.

12. The method of claim 10, wherein the interior surface of the one or more microfluidic channels is coated with a plurality of endothelial cells.

13. The method of claim 10, wherein the one or more microfluidic channels comprises at least one inlet and an outlet.

14. The method of claim 10, wherein a lensless in-line digital holography configuration is used for imaging.

15. The method of claim 10, wherein the plurality of blood cells are unstained and untagged.
